(19) Europäisches Patentamt
European Patent Office
Office européen des brevets

(11) Veröffentlichungsnummer: **0 154 937**
**A2**

(12) # EUROPÄISCHE PATENTANMELDUNG

(21) Anmeldenummer: **85102571.8**

(22) Anmeldetag: **07.03.85**

(51) Int. Cl.⁴: **C 07 D 473/34**, A 01 N 43/90

(30) Priorität: **14.03.84 DE 3409272**

(43) Veröffentlichungstag der Anmeldung: **18.09.85 Patentblatt 85/38**

(84) Benannte Vertragsstaaten: **BE CH DE FR GB IT LI NL**

(71) Anmelder: **BASF Aktiengesellschaft, Carl-Bosch-Strasse 38, D-6700 Ludwigshafen (DE)**

(72) Erfinder: **Bliesener, Jens-Uwe, Dr., Silvanerweg 16, D-6705 Deidesheim (DE)**
Erfinder: **Sauter, Hubert, Dr., Neckarpromenade 20, D-6800 Mannheim 1 (DE)**
Erfinder: **Goetz, Norbert, Dr., Schoefferstrasse 25, D-6520 Worms 1 (DE)**
Erfinder: **Jung, Johann, Prof. Dr. Dipl.-Landwirt, Hardenburgstrasse 19, D-6703 Limburgerhof (DE)**
Erfinder: **Grossmann, Klaus, Dr., Buschstrasse 34, D-6708 Neuhofen (DE)**

(54) **6-(3-Thienylmethylamino)-purin, Verfahren zu seiner Herstellung und seine Verwendung.**

(57) 6-(3-Thienylmethylamino) -purin der Formel I

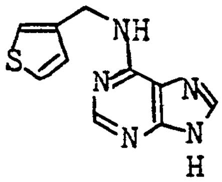

(I)

Verfahren zu seiner Herstellung sowie Mittel zur Regulierung des Pflanzenwachstums, enthaltend die Verbindung der Formel I.

EP 0 154 937 A2

6-(3-Thienylmethylamino)-purin, Verfahren zu seiner Herstellung und seine Verwendung

Die Erfindung betrifft das neue 6-(3-Thienylmethylamino)-purin (auch: Thenylaminopurin) der Formel I, Verfahren zu seiner Herstellung und seine Verwendung zur Regulierung des Pflanzenwachstums.

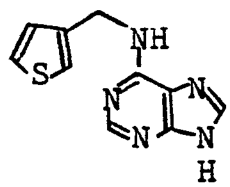
(I)

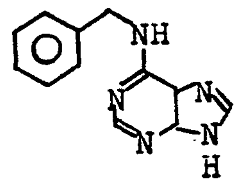
(II)

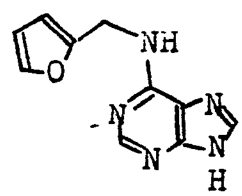
(III)

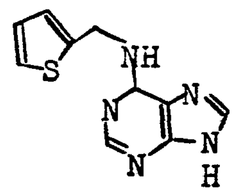
(IV)

Es ist bekannt, daß 6-Benzylaminopurin der Formel II ("Benzyladenin") ähnlich dem natürlichen Phytohormon 6-(2-Furylmethylamino)-purin der Formel III ("Kinetin") das Pflanzenwachstum beeinflußt (siehe z.B. Wegler, Chemie der Pflanzenschutz- und Schädlingsbekämpfungsmittel).

Als Beispiele für die physiologischen Wirkungen sind zu nennen: die Steuerung des Zellteilungswachstums in Callusgeweben und intakten Pflanzen, die Förderung der Knospen- und Blütenbildung, Stimulierung der Samenkeimung, Überwindung der Ruhephasen von Samen, Speicherorganen und Knospen, Förderung der Seitentriebbildung und Verzögerung der Alterungsprozesse in Pflanzen.

Man spricht in diesem Zusammenhang von cytokininartigen Wirkungen.

Auch das 6-(2-Thienylmethylamino)-purin der Formel IV besitzt cytokininartige Wirkungen, die jedoch schwächer ausgeprägt sind als diejenigen von Benzyladenin der Formel II ( Phytochemistry 16, 1865 (1977)). Die Aufgabe besteht daher, Wirkstoffe zu finden, die die Wirkung der bekannten Adeninabkömmlinge übertreffen.

Es wurde nun gefunden, daß das zu IV isomere 6-(3-Thienylmethylamino)--purin der Formel I hervorragende pflanzenwachstumsregulierende Eigenschaften besitzt, die auch derjenigen des Benzyladenins deutlich überlegen sind.

Der erfindungsgemäße Wirkstoff (I) kann z.B. dadurch hergestellt werden, daß man 6-Aminopurin mit Thiophen-3-aldehyd in Gegenwart von Ameisensäure reduktiv alkyliert. Eine andere Möglichkeit der Herstellung besteht darin, daß man 6-Aminopurin zunächst mit Thiophen-3-carbonsäurechlorid acyliert und anschließend zur Verbindung der Formel I reduziert. Weitere denkbare Herstellmöglichkeiten hängen u.a. von der Verfügbarkeit entsprechender Rohstoffe ab; auch können die erzielbaren Ausbeuten oder der wirtschaftliche Aufwand für die Ausgestaltung des Herstellverfahrens in technischem Maßstab unterschiedlich sein. Eine Untersuchung mit den fachüblichen Mitteln der physikalischen Chemie und der Verfahrenstechnik ist daher zu empfehlen.

Für die erstgenannte Verfahrensvariante wird ein Moläquivalent Adenin mit 1 bis 50, bevorzugt 2 bis 20 Moläquivalenten Ameisensäure sowie mit 0,5 bis 2, bevorzugt 0,9 bis 1,2 Moläquivalenten Thiophen-3-aldehyd vorteilhaft in Anwesenheit eines Lösungsmittels auf etwa 90 bis 120°C (z.B. auf Rückflußtemperatur) erhitzt. Die Aufarbeitung und Reinigung des Rohgemisches erfolgt in üblicher Weise z. B. durch Abdestillieren überschüssiger Reagenzien, extraktive Reinigungsschritte und ggf. Umkristallisieren, etwa wie in Beispiel 1 beschrieben.

<u>Beispiel 1</u>
Herstellung von 6-(3-Thienylmethylamino)-purin; Verfahrensvariante A

405 g (3 mol) Adenin werden unter Eiskühlung in 1 700 ml (45 mol) Ameisensäure gelöst und dann mit 504 g (4,5 mol) Thiophen-3-aldehyd versetzt. Man erhitzt 15 Stunden am Rückfluß. Anschließend wird die überschüssige Ameisensäure langsam während 8 Stunden abdestilliert, wobei gegen Ende die Temperatur bis auf etwa 180°C steigt. Nach dem Abkühlen gibt man zum Rückstand 1,5 l 25 % wäßrige Natriumhydroxidlösung und rührt die Mischung über Nacht. Sodann wird vom Ungelösten abfiltriert und das Filtrat dreimal mit je 1 l Dichlormethan extrahiert. Die wäßrige Phase wird mit 10prozentiger Salzsäure auf pH 8 bis 9 eingestellt, wobei das Zielprodukt ausfällt. Nach dem Abfiltrieren, Waschen mit Wasser und Trocknen bei 60°C unter vermindertem Druck erhält man 687 g 6-(3-Thienylmethylamino)-purin; Fp. 227 - 229°C.

Für die andere Verfahrensvariante wird ein Moläquivalent Adenin in an sich bekannter Weise (Phytochemistry <u>16</u>, 1865 (1977)) mit 1 bis 10, bevorzugt 2 bis 5 Moläquivalenten Thiophen-3-carbonsäurechlorid in Gegenwart eines Protonenfängers (z.B. Pyridin) eventuell in einem Lösungsmittel wie Dioxan auf bis zu 200°C erhitzt und in üblicher Weise aufgearbeitet und ggf. umkristallisiert, etwa wie oben beschrieben.

Die Reduktion des so erhaltenen 6-(3-Thenoylamino)-purin (auch: Thienylcarbonylaminopurin) kann man in bekannter Weise, z.B. in einem Lösungsmittel bis zu 200°C, bevorzugt 50 bis 100°C mit einem Reduktionsmittel (z.B. Diboran in Gegenwart einer Lewis-Säure oder Lithiumaluminiumhydrid) vornehmen und wie üblich aufarbeiten. Besonders glatt verläuft die Reduktion mit Lithiumaluminiumhydrid in Tetrahydrofuran etwa wie in Beispiel 2 b beschrieben.

Beispiel 2

Herstellung von 6-(3-Thienylmethylamino)-purin; Verfahrensvariante B

a) Zu einer Suspension von 19,7 g (0,146 mol) Adenin in 100 ml trockenem Pyridin werden 62,6 g (0,439 mol) Thiophen-3-carbonsäurechlorid gegeben. Man erhitzt 10 Stunden am Rückfluß, destilliert das Pyridin ab, nimmt in Chloroform auf und wäscht mit verdünnter Natriumhydrogencarbonatlösung. Nach Trocknen über Natriumsulfat wird das Lösungsmittel im Rotationsverdampfer abgezogen. Der feste Rückstand wird wiederholt mit Cyclohexan gerührt, anschließend aus Ethanol umkristallisiert und bei ca. 40°C i.V. getrocknet. Man erhält 6-(3-Thenoylamino)-purin in einer Ausbeute von 60 %.

b) Zu einer Suspension von 6,5 g (0,17 mol) Lithiumaluminiumhydrid in 350 ml absolutem Tetrahydrofuran wird unter Rühren bei 80°C eine Suspension von 20 g (0,082 mol) 6-(3-Thenoylamino)-purin in 1 l absolutem Tetrahydrofuran zugetropft. Nach 3 Stunden wird der Hydrid-Überschuß mit Natriumsulfat-Decahydrat zersetzt und der Rückstand abfiltriert. Man bringt zur Trockene und kristallisiert den erhaltenen öligen Kristallbrei mehrmals aus Tetrahydrofuran. Man erhält 6-(3-Thienylmethylamino)-purin in einer Ausbeute von 25 %.

Der neue Wirkstoff greift in den Metabolismus der Pflanzen ein und kann deshalb als Wachstumsregulator eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z. B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten des erfindungsgemäßen Pflanzenwachstumsregulators im Pflanzenbau, in der Landwirtschaft und im Gartenbau, werden einige nachstehend erwähnt.

A. Man kann das vegetative Wachstum vieler Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z. B. die Verringerung des Grasbewuchses an Straßenrändern, Kanalböschungen und auf Rasenflächen wie Park-, Sport- udn Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung des Wachstumsregulators kann auch die seitliche Verzweigung der Pflanzen vermehrt oder gehemmt werden. Daran besteht

Interesse, wenn z. B. bei Tabakpflanzen die Ausbildung von Seitentrieben (Geiztrieben) zugunsten des Blattwachstums gehemmt werden soll.

Eine Möglichkeit der Ertragssteigerung mit Hilfe des Wirkstoffs beruht darauf, daß die Nährstoffe in stärkerem Maße der Blüten- und Fruchtbildung zugute kommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag bezogen auf die Bodenfläche erzielt werden kann. Die erfindungsgemäße Verbindung eignet sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit dem neuen Wirkstoff lassen sich insbesondere Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

So können Ertragssteigerungen durch Eingriffe in den pflanzlichen Stoffwechsel bzw. durch Förderung oder Hemmung des vegetativen und/oder des generativen Wachstums erreicht werden.

C. Mit dem Wirkstoff lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der Pflanze ist auch für ein gut kontrollierbares Entblättern von Bäumen oder Sträuchern wesentlich. So läßt sich mit der erfindungsgemäßen Verbindung Baumwolle defoliieren und damit die mechanische Ernte erleichtern.

Die Wirkung der neuen Verbindung ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotylen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Der Wirkstoff kann den Kulturpflanzen auf dem Weg über das Saatgut, d.h. als Saatgutbeize, über den Boden, oder durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha ausreichend.

Das Mittel kann die Form einer üblichen Formulierung haben, also z.B. eine Lösung, Emulsion, Suspension, Staub, Pulver, Paste oder ein Granulat sein. Die Anwendungsform richtet sich nach dem Verwendungszweck; sie soll in jedem Fall eine feine und gleichmäßige Verteilung der wirksamen Substanz gewährleisten. Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole, Paraffine (z. B. Erdölfraktionen), Alkohole (z. B. Methanol, Butanol), Amine (z. B. Ethanolamin), Ketone (z. B. Cyclohexanon), Dimethylformamid und Wasser; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel oder sonstige oberflächenaktive Mittel, wie nichtionogene und anionische

Emulgatoren (z. B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindung als wäßrige Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95, vorzugsweise zwischen 50 und 90 Gewichtsprozent Wirkstoff.

Die Anwendung kann zeitlich in jeder Weise, beispielsweise im Vorauflaufverfahren, im Nachauflaufverfahren oder als Beizmittel geschehen.

In den nachfolgenden Beispielen wird die Wirkung des Wirkstoffs als Pflanzenwachstumsregulator dargestellt, ohne die Möglichkeit einer anderen Anwendung als der eines Wachstumsregulators auszuschließen.

Beispiel 1

Zunahme des Frischgewichts von Radieschen-Kotyledonen (nach Letham 1971, Physiol. Plant. 25, 391-396)

Von gleichmäßig großen, frisch gekeimten Radieschensamen (Sorte Karissima GS) wurde das äußere Kotyledon der Keimlinge geerntet. Jeweils 6 Kotyledonen wurden in Glaspetrischalen (5 cm Ø) mit 2 ml 1 mM Kaliumphosphatpuffer pH 6,0, der die zu untersuchende Probe enthielt, inkubiert. Nach 72-stündiger Inkubation bei 25°C im Dunkeln wurde die Frisch-

gewichtszunahme der Kotyledonen als Maß für die Wirkung der Pflanzenwachstumsregulatoren ermittelt. Angegeben werden die Mittelwerte aus parallelen Ansätzen (Tabelle 1).

Tabelle 1

| molare Konzentration | Zunahme des Frischgewichts in % zur Kontrolle | | | |
|---|---|---|---|---|
| | 6-(3-Thienyl-methylamino)-purin | Benzyl-adenin | Kinetin | Isopentenyl-adenin |
| $10^{-4}$ | 262 | 236 | 200 | 227 |
| $10^{-5}$ | 220 | 203 | 207 | 142 |
| $10^{-7}$ | 189 | 185 | 151 | 82 |

Aus der Tabelle 1 geht hervor, daß bei der Behandlung von Radieschen--Kotyledonen mit dem erfindungsgemäßen Mittel höhere Frischgewichtszunahmen als mit den bekannten Pflanzenwachstumsregulatoren mit cytokininartiger Wirkung erzielt wurde.

Beispiel 2

Induzierung der Knospenbildung am Funaria-Vorkeim (Hahn und Bopp 1968, Planto 83, 115-118)

Sporenkapseln des Laubmooses Funaria hygrometrica (L.) Sibth. wurden unter aseptischen Bedingungen geöffnet und Protonema Kulturen aus den einzelnen Sporen auf der Oberfläche eines Knop-Agars bei 21°C und eine Lichtintensität von 2000 Lux angezogen. Nach 4 bis 5 Tagen wurden einzelne Protosemata isoliert und in eine neue Petrischale mit Knop-Agar und Cellophanauflage transferiert. Nach weiteren 8 bis 11 Tagen wurden 10 Caulonema-Filamente isoliert, auf der Cellophanoberfläche nebeneinander aufgereiht, die Streifen ausgeschnitten und auf Agarplatten mit Wirkstoffzusatz übertragen. Nach 2 bis 3 Tagen wurde die Anzahl der induzierten Knospen an den Caulonemafäden als Maß für die cytokininartige Wirkung der Pflanzenwachstumsregulatoren ermittelt (Tabelle 2).

Tabelle 2

| molare Konzentration | Anzahl der induzierten Knospen über die Kontrolle 6-(3-Thienylmethylamino)-purin | Benzyladenin | Kinetin | Isopentenyladenin |
|---|---|---|---|---|
| $10^{-4}$ | 62 | 38 | 34 | - |
| $10^{-5}$ | 60 | 43 | 8 | 37 |

Die Tabelle 2 zeigt, daß das erfindungsgemäße Mittel weitaus die größte Anzahl neuer Knospen induziert.

Beispiel 3

Hemmung der Blattalterung bei Gerste (Großmann und Jung 1982, Z.Acker- und Pflanzenbau, 151, 149-165)

Als Versuchspflanzen dienten 12 bis 14 Tage alte Gerstenpflanzen (Sommergerste, Sorte Union), die unter Gewächshausbedingungen angezogen wurden. Das Primärblatt wurde abgetrennt und in 1 cm lange Segmente zerteilt. Sechs Blattsegmente wurden in je eine sterile Petrischale (4 cm ∅) mit 2 ml sterilem destilliertem Wasser und unterschiedlicher Zugabe von Wirkstoffen überführt. Nach 3 Tagen Inkubation bei 25°C im Dunkeln wurden die Blattsegmente der Parallelansätze vereinigt und in einer Reibschale unter flüssigem $N_2$ gemörsert. Das Homogenat wurde mit 1 ml 25 mM Tris-HCl--Puffer (pH 7,5) und einer Spatelspitze Kollidon versetzt und kurz zentrifugiert. Der Überstand wurde zur Bestimmung des löslichen Proteingehalts über Komplexbildung mit dem Farbstoff Coomassie Brilliant Blue G 250 und photometrische Auswertung bei 595 nm herangezogen. Der Chlorophyllgehalt der Blattsegmente wurde über photometrische Auswertung bei 652 nm der methanolischen Auszüge des Sediments ermittelt (Tabelle 3).

Tabelle 3

| Verbindung ($10^{-4}$M) | 6-(3-Thienyl-methylamino)-purin | Benzyl-adenin | Kinetin | Isopentenyl-adenin |
|---|---|---|---|---|
| Chlorophyll-Gehalt | 170 | 165 | 154 | 160 |
| Protein-Gehalt | 140 | 125 | 130 | 133 |

Werte in % zur Kontrolle

Die Tabelle 3 zeigt, daß das erfindungsgemäße Mittel sowohl den Abbau des Chlorophylls als auch des löslichen Proteins der Gerstenblattsegmente bedeutend hemmte.

Beispiel 4

Induzierung von Seitentrieben bei Vicia faba

8 Tage alte Ackerbohnen-Keimlinge (Sorte Herra) wurden unter Gewächshausbedingungen angezogen und anschließend in Hydrokultur gehalten. Die Keimlinge wurden dafür am Wurzelhals mit Schaumstoff umwickelt und in Deckelöffnungen von zylindrischen Kunststoffgefäßen (Volumen 4 l) eingepaßt. Die Nährlösung nach Linsmaier und Skoog (1964), Physiol. Plant. 18, 100-127) enthielt die einzelnen Wirkstoffe in den angegebenen Konzentrationen. Nach 4 Wochen wurde die Anzahl der induzierten Seitentriebe als Maß für die cytokininartige Wirkung der Pflanzenwachstumsregulatoren ermittelt (Tabelle 4).

Tabelle 4

| molare Konzentration | Anzahl der Seitentriebe / 5 Pflanzen | | | |
|---|---|---|---|---|
| | 6-(3-Thienyl-methylamino)-purin | Benzyl-adenin | Kinetin | Kontrolle |
| $10^{-5}$ | 7 | 3 | 1 | 0 |
| $10^{-6}$ | 3 | 1 | 0 | 0 |

Aus der Tabelle 4 geht hervor, daß mit dem erfindungsgemäßen Mittel weitaus die meisten Seitentriebe induziert werden konnten, was auf eine deutliche Verminderung der apikalen Dominanz der Ackerbohnenpflanzen hervorgerufen durch das erfindungsgemäße Mittel hinweist.

Beispiel 5
Reduzierung der Wuchshöhe von Sojabohne

8 Tage alte Sojabohnen-Keimlinge (Sorte Gieso) wurden unter Gewächshausbedingungen angezogen und anschließend in Hydrokultur gehalten. Die Nährlösung nach Linsmaier und Skoog enthielt die einzelnen Wirkstoffe in den angegebenen Konzentrationen. Nach 3 Wochen wurde die beobachtete wachstumsregulierende Wirkung durch Wuchshöhenmessung belegt. Die so gewonnenen Meßwerte wurden zur Wuchshöhe der unbehandelten Pflanzen (Kontrolle) in Relation gesetzt.

Parallel zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind Tabelle 5 zu entnehmen.

Tabelle 5

| molare Konzentration | Wuchshöhen (in % zur Kontrolle) 6-(3-Thienylmethylamino)-purin | Benzyladenin | Kinetin |
|---|---|---|---|
| $10^{-5}$ | 48 | 50 | 54 |
| $10^{-6}$ | 47 | 54 | 72 |

Die Tabelle 5 zeigt, daß das erfindungsgemäße Mittel am deutlichsten die Wuchshöhe von Sojapflanzen reduziert.

0154937

Patentansprüche

1. 6-(3-Thienylmethylamino)-purin der Formel I

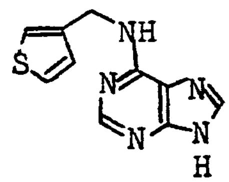

(I)

2. Verfahren zur Herstellung der Verbindung gemäß Anspruch 1, dadurch gekennzeichnet, daß man Adenin mit Thiophen-3-aldehyd in Gegenwart von Ameisensäure reduktiv alkyliert.

3. Verfahren zur Herstellung der Verbindung gemäß Anspruch 1, dadurch gekennzeichnet, daß man Adenin mit Thiophen-3-carbonsäurechlorid acyliert und das entstandene Amid anschließend reduziert.

4. Mittel zur Regulierung des Pfanzenwachstums, enthaltend die Verbindung gemäß Anspruch 1.

5. Mittel zur Regulierung des Pflanzenwachstums, enthaltend die Verbindung gemäß Anspruch 1 und einen flüssigen oder festen Trägerstoff sowie gegebenenfalls ein oder mehrere oberflächenaktive Mittel.

6. Verwendung der Verbindung oder des Mittels gemäß Anspruch 1 bzw. 4 oder 5 zur Regulierung des Pflanzenwachstums.

7. Verfahren zur Regulierung des Pflanzenwachstums, dadurch gekennzeichnet, daß man die Verbindung gemäß Anspruch 1 auf Pflanzen, Pflanzensamen oder deren Lebensraum einwirken läßt.

8. Verfahren zur Herstellung von das Pflanzenwachstum regulierenden Mitteln, dadurch gekennzeichnet, daß man die Verbindung gemäß Anspruch 1 mit mindestens einem festen oder flüssigen Trägerstoff sowie gegebenenfalls einem oder mehreren oberflächenaktiven Mitteln mischt.